# EUROPEAN PATENT APPLICATION

(11) **EP 4 692 067 A1**
(43) Date of publication of application: **11.02.2026**
(21) Application number: 24785432.6
(22) Date of filing: 06.04.2024
(51) Int. Cl.: C07D 239/42, C07D 401/04, C07D 403/04, C07D 413/04, C07C 27/02, C07C 51/41, A01N 43/54, A01N 43/60, A01N 43/84, A01P 21/00

(54) **SALTS OF N-SUBSTITUTED 2-AMINO-4-METHYLPYRIMIDINE-5-YL-CARBOXYLIC ACIDS AS GROWTH STIMULATORS FOR PLANTS AND FUNGI**

(30) Priority: 07.04.2023 RU 2023108799
(71) Applicant: OCC Management GmbH, 6300 Zug (CH)
(72) Inventor: POTAPOV, Andrey Iurievich, Voronezh, 394031 (RU)
(74) Representative: Rentsch Partner AG
(86) International application number: PCT/RU2024/050076
(87) International publication number: WO 2024/210773

(57) **Abstract**

The invention relates to novel salts of N-substituted 2-amino-4-methylpyrimidine-5-yl-carboxylic acids having the formula (I), where the values of the radicals are disclosed in the claims, and the use of said salts as a growth stimulator for plants and fungi. The invention further relates to embodiments of methods for producing N-substituted 2-amino-4-methylpyrimidine-5-ylcarboxylic acids having the formula (I). The technical result is the production of novel salts of N-substituted 2-amino-4-methylpyrimidine-5-yl-carboxylic acids having the formula (I) which exhibit improved plant growth stimulator properties.

## Description

### Technical field

This invention relates to novel compounds intended for use in agriculture, particularly for regulating of growth and development of plants, and could be used to exert a stimulatory effect on plants and fungi.

In particular, these new compounds can be used to optimize plant growth parameters such as germination energy, seed germination rate, biometric characteristics of seedlings, root formation, and yield across a wide range of agricultural crops.

### Prior art

Plant cultivation faces numerous problems regarding extreme temperature excursions, lack of moisture and poor climate conditions in some regions. Thus, new means are required for stimulation of plants growth and development, which can be achieved through the use of synthesized chemical compounds. (Vasin, A.V. Primeneniye stimuliatorov rosta pri vyrashcivanii kukuruzy i yachmenya [Use of growth stimulator in growing of maize and barley] A.V. Vasin, A.V. Darmin, V.V. Brezhnev / Kormoproisvodstvo. - 2009. - No. 2. - pp. 17-18; Vasin, V.G. Effektivnost primeneniya stimuljatora rosta pri vyrashchivanii kukuruzy na zerno [Efficiency of the growth stimulator during maize cultivation for grain] / V.G. Vasin, A.V. Darmin, A.V. Vasin / Izvestia samarskoy gosudarstvennoy selskohozyastvennoy akademii. - 2008. - No. 4. - pp. 22-24; Ostroshenko, V.V. Vliyanie predposevnoy obrabotki semyan stimuljatorami rosta na ikh posevnye kachestva [Effect of re-sowing treatment with growth stimulators on their sowing qualities] / V.V. Ostroshenko, L. Yu. Ostroshenko / Vestnik Krasnoyarskogo gosudarstvennogo agrarnogo universiteta. - 2011. - No. 5. - pp. 12-15; Shishov A.D. Opredeleniye rostostimuliruyushchikh koncentraciy novykh reguliatorov rosta s induktorov ustoichivosti rasteniy [Determination of growth stimulating concentrations of new growth stimulators and plant resistance inducers]. A.D. Shishov, G.L. Matevosian / Fundamentalnyie issledovaniya. - 2005. - No. 9. - pp. 46-47; 16; Sinkov A.A. Vlyyanie regulyatorov rosta na organichenie abioticheskikh i bioticheskikh stressov pri vyrashchivanii ozymoy pshenitsy na vyshchelochennom chernozeme yuga Nechernozem'ya [Effect of growth regulators on restriction of abiotic and biotic stresses during cultivation of the winter wheat on the leached chernozem of the South Non-Black Soil Belt]: Autoref. dis. kand. s/kh nauk / A.A. Sinkov. - Saratov, 2011 - 21 pp. Vlyyanie sinteticheskogo regulyatora rosta tsitodef y tyazhelykh metallov na okislitelny status rasteniy ogurtsa [Effect of synthetic growth regulator citodef and heavy metals on the oxidative status of the cucumber plants] D.I. Bashmakov i [dr] / Fiziologiya rasteniy. - 2012. - V. 59, No. 1. - pp. 67-73).

The patent RU 2485083 C1 describes substituted pyrimidine-5-carboxylic acids of general formula 1, which are structurally similar to the compounds proposed herein. 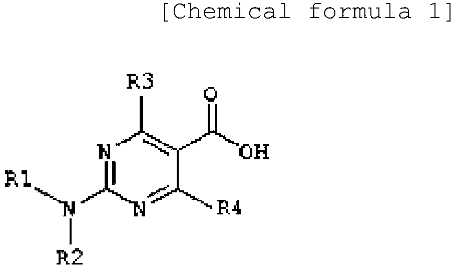 where R₁+R₂ = piperidine, pyrrolidine, morpholine, N-Alk(Ar) piperazine, or R₁ = Alk, Ar; R₂ = Alk, Ar or R₁ = H, R₂ = H, Alk, Ar, Het; R₃ = Alk, particularly methyl, Ar etc., R₄ = H. In particular, there are compounds described where R₁+R₂ = piperidine or R₁ = hydrogen, R₂ = phenyl, 2-benzyl, 2-phenylethyl, 4-methylphenyl, benzoxazol-2-yl, benzothyazol-2-yl, 4-methylquinazol-2-yl; R₃- methyl R₄-hydrogen. The compounds exhibit growth-regulating activity with respect to French marigold (Tagetes patula L).

The known compounds for the formula (1) were obtained according to the following scheme:

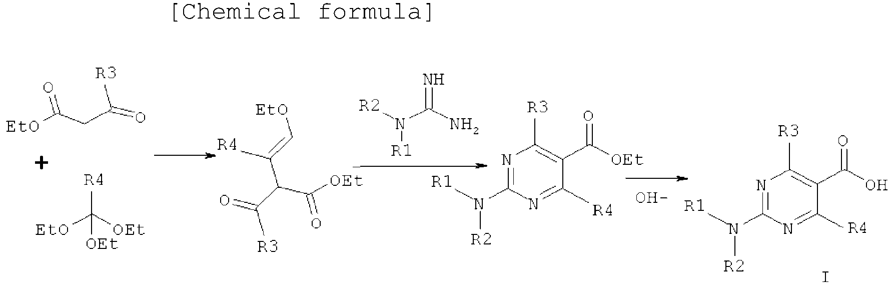

Patent RU 2788114 C1 describes application of the compound corresponding to the aforementioned general formula (1), where R₁ = hydrogen, R₂ = benzyl, R₃ = methyl, R₄ = hydrogen, as the growth stimulator for the sugar beet.

Patent RU 2490893 C1 describes a method for using compound corresponding to the aforementioned general formula (1), where R₁ = hydrogen, R₂ = benzyl, R₂ = methyl, R3=hydrogen, for the stimulation of growth for French Marigold.

Patent RU 2663068 C1 describes a method for stimulating the growth of plant species of the genus Rhododendron L., using compounds of formula (A), where R₁ = hydrogen, R₂ = benzyl, or R1 and R2 together with the nitrogen atom represent piperidine, and R₃ = methyl.

Patent RU 2656393 C1 describes a method for using compounds corresponding to the aforementioned general formula (1), where R₁ = hydrogen, R₂ = benzyl, or R₁ and R₂ together with the nitrogen atom means piperidine, morpholine, for stimulation of growth in plants of the genus Rhododendron L.

Patent RU 2659828 C1 describes a method for using compounds of formula (1), where R₁ and R₂ together with the nitrogen atom means piperidine, morpholine, R₃ is methyl, for stimulation of growth for annual Salvia splendens.

Patent RU 2678119 C1 describes a method for using compounds of formula (1), where R₁ = hydrogen, R₂ = benzyl, or R₁ and R₂ together means piperidine, morpholine, R₃ is methyl, for stimulation of growth for common tomato plant (Solarium lycopersicum L).

However, known substituted pyrimidine-5-carboxylic compounds exhibit insufficient seed germination, total yield and plant height, and have a significant drawback related to their very poor solubility in water, which complicates their dosage and application.

Patent US 3040047 B1 (example 17, columns 6, 7) describes the method of obtaining sodium salt
of 2-(4-5-dimethyl-1H-pyrazol-1yl)-4-methylpyrimidine-carboxylic acid (1:1). The method involves adding sodium hydroxide solution and ethanol to the
2-(4-5-dimethyl-1H-pyrazol-1-yl)-4-methyl-5-carbetoxypyrimidine. The mixture is boiled, then concentrated, and crystalline sodium salt is isolated. The known salt is used as an intermediate for subsequent production of correspondent carboxylic acid.

The salts of this invention do not contain the 4,5-dimethyl-1H-pyrazol-1-yl group in the position 2 of pyrimidine ring, and have different purpose - they are used for plant growth stimulation.

### Summary of the invention

### Technical problem

The technical problem of this invention is to improve the physicochemical properties of plant growth stimulants, in particular to increase their solubility, as well as to enhance seed germination, plant height, biomass, and yield when the growth stimulant is applied during pre-sowing seed treatment or at any stage of plant development.

### Solution to the problem

The problem concerned is solved by using new plant growth stimulators, i.e. salts of the N-substituted 2-amino-4-methylpyrimidine-5-yl-carboxylic acids of the general formula (I): 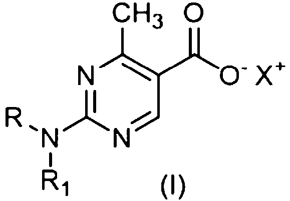
where X⁺ is selected from cations such as Na⁺, K⁺, Li⁺, ½ Mg²⁺, ½ Ca⁺, NH₄⁺, H₃N⁺CH₂CH₂OH, H₃N⁺CH₂CH₂CH₂OH, HN⁺(CH₂CH₂OH)₃, H₃N⁺CH₂CH₂C₆H₅, or ANH₃⁺, A₂NH₂⁺, A₃NH⁺,
A is alkyl C1-C12, aryl C6-C12, heterocycle C5-C12, heteroaryl C5-C12, where heterocycle and heteroaryl compounds contain 1 to 3 hetero atoms, which could be N, O or S,
while each A may be optionally substituted by one or several groups, which independently could be selected from halogen, amino- or thio- groups.

The term "alkyl" includes linear, branched or cyclic hydrocarbon structures and their combinations, with carbon chain length varying from 1 to 12 C atoms, preferably from 1 to 6 carbon atoms, more preferably from 1 to 3 carbon atoms.

The term "aryl" means phenyl, biphenyl or naphtyl groups.

The term "heteroaryl", used alone or in combination, means 5 to 10-member monocyclic or bicyclic aromatic ring that contains 1, 2 or 3 heteroatoms, which can be independently selected from oxygen, nitrogen or sulphur. Preferably, the term "heteroaryl" means 5 to 10-member monocyclic or bicyclic aromatic ring that contains 1 nitrogen atom, and optionally, one extra heteroatom, which can be independently selected from oxygen, nitrogen or sulphur.

Examples of such heteroaryl groups include furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzoisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, cinnolinyl, quinazolinyl, quinoxalinyl, and phthalazinyl.

In the preferred variant, R₁ means methyl, ethyl, propyl, phenyl, optionally substituted by one or two substituting groups selected from methyl, ethyl, methoxy group, halogen; and phenyl-(C₁-C₂)alkyl-.

In the preferred variant, R+R₁ together with the nitrogen atom mean pyrrolydine, hydroquinoline, hydroisoquinoline, piperidine, morpholine, piperazine, optionally substituted by one or two substituting groups selected from methyl, ethyl, halogen and phenyl.

In the preferred variant, cation is either Na⁺, or K⁺, Li+, ½ Mg²⁺, ½ Ca+, NH₄⁺, H₃N⁺CH₂CH₂OH, H₃N⁺CH₂CH₂CH₂OH, H₃N⁺CH₂CH₂C₆H₅, HN⁺(CH₂CH₂OH)₃.

The proposed salts, their preparation, and their use for stimulating plant growth have not been previously known.

The plants could be common tomato (Solarium lycopersicum L), sugar beet, annual Salvia splendens, French marigolds, plants of genus Rhododendron L. etc.

Salts of N-substituted 2-amino-4-methylpyrimidine-5-yl-carboxylic acids of formula (I) demonstrate good solubility in water, and their solutions may be used in the agriculture for pre-sowing seed treatment, and also for subsequent application to the soil in various stages of plants and fungi development, both individually and in combination with other substances as part of premixes.

Salts of N-substituted 2-amino-4-methylpyrimidine-5-yl-carboxylic acids of formula (I) are convenient and efficient when used as plant growth stimulators.

As salts of N-substituted 2-amino-4-methylpyrimidine-5-yl-carboxylic acid of formula (I), in particular, the salts of the following acids may be used: 2-(2-phenylethyl)amino-4-methylpyrimidine-5-carboxylic acid, 4-methyl-2-(pyrrolidin-1-yl)pyrimidine-5-carboxylic acid, 4-methyl-2-(N-methylpiperazin-1-yl)pyrimidine-5-carboxylic acid, 4-methyl-2-((4-methoxyphenyl)amino)pyrimidine-5-carboxylic acid, 2-benzylamino-4-methylpyrimidine-5-carboxylic acid, 2-(3,4-dihydroisoquinolin-2(1H)-yl)-4-methylpyrimidine-5-carboxylic acid, 4-methyl-2-((2,4-difluorophenyl)amino)pyrimidine-5-carboxylic acid, 4-methyl-2-((4-methylphenyl)amino)pyrimidine-5-carboxylic acid, 4-methyl-2-(morpholin-4-yl)pyrimidine-5-carboxylic acid, 4-methyl-2-(piperidin-1-yl)pyrimidine-5-carboxylic acid, 4-methyl-2-((3-methylphenyl)amino)pyrimidine-5-carboxylic acid, etc.

### Advantages of the invention

During investigation, comparative assessment was performed for the effect of the salts of N-substituted 2-amino-4-pyrimidine-5-yl-carboxylic acid and related N-substituted 2-amino-4-pyrimidine-5-yl-carboxylic acid on morphometric indices such as seed germination rate, yield, plant and roots height.

The plants selected for tests were common tomato, Novichok cultivar (Solarium lycopersicum L.). The following seeds and plant materials were used: seeds from the company Aelita (Moscow); common aubergine (Solanum melongena L.), Sirenevyy cultivar; perennial Rhododendron Ledebourii Pojarc.; French marigold (Tagetes patula L.), Krasnaya Vishnya cultivar; winter wheat (Triticum aestivum L.), Alekseich cultivar; potato (Solanum tuberosum), Zekura cultivar; sunflower (Helianthus L.), Chakinskiy 100 cultivar; maize hybrid (Zea mays L.), "Mixi"; zucchini (Cucurbita pepo subsp. pepo), Tintoretto cultivar; pea (Pisum L.), Orlus cultivar; white cabbage (Brassica oleracea L.), Podarok cultivar; sweet pepper (Capsicum annuum L.); two-spore white button mushroom.

The technical effect was achieved during the planting of seeds (or mycelium) by single treatment of soil with aqueous suspensions of the following compounds: 2-(2-phenylethyl)amino-4-methylpyrimidine-5-carboxylic acid, 4-methyl-2-(morpholin-1-yl)pyrimidine-5-carboxylic acid, 4-methyl-2-(N-methylpiperazin-1-yl)pyrimidine-5-carboxylic acid, 2-benzylamino-4-methylpyrimidine-5-carboxylic acid, 4-methyl-2-(piperidin-1-yl)pyrimidine-5-carboxylic acid, 4-methyl-2-((2,4-difluorophenyl)amino)pyrimidine-5-carboxylic acid, 4-methyl-2-((4-methylphenyl)amino)pyrimidine-5-carboxylic acid, 4-methyl-2-(morpholin-4-yl)pyrimidine-5-carboxylic acid, 4-methyl-2-(piperidin-1-yl)pyrimidine-5-carboxylic acid, 2-(3,4-dihydroisoquinolin-2(1H)-yl)-4-methylpyrimidine-5-carboxylic acid, 4-methyl-2-((3-methylphenyl)amino)pyrimidine-5-carboxylic acid, and this was compared with aqueous solutions of the salts of the corresponding acids as proposed in this invention. Application rate was 0.0002 g/m² (2 g/ha). In the control tests, seedlings were irrigated with water. The results of effect of various preparations are presented in the tables 1-13.

### Description of embodiments

In order to study effect of N-substituted 2-amino-4-pyrimidine-5-yl-carboxylic acids and their salts on seed germination, the seeds were soaked in 0.0015% (w/w) solution with the exposure time of 2-18 hours, depending on the treated crop. As the control group, the seeds soaked in the tap water were used, but in case of tomato, also seeds soaked in commercially available growth regulator Epin Extra (manufactured by NNPP NEST M, RF, hereinafter "Epin"), in the concentration as per IFU, i.e. 0.05%). Each experiment was performed in three replicates for each tested substance and the control group, using 200 seeds or the amount required for field trials.

Seeds were germinated at 23 °C. The seedlings were planted on the 15th day after the start of germination. The germination rate was calculated as a percentage ratio of the number of seedlings to the number of the seeds used.

The next experiment was conducted according to B.A. Dospekhov's method (Metodika polevogo opyta (s osnovami statisticheskoy obrabotki resultatov issledovaniy [Field trial methodology (including the basics of statistical analysis of the experimental results)]) 5-th edition, revised and amended. Higher education textbook / B.A. Dospekhov. Moscow: Agropromizdat publ., 1985 - 351 pp) of split plots in triplicate or on several fields in case of a field trial.

Results were pooled and processed as per method stated in A.P. Kulaichev's work (Metody i sredstva kompleksnongo anlalyza dannykh [Methods and tools for comprehensive data analysis] / A.P. Kulaichev. Moscow: FORUM: INFA publ., Moscow, 2006 - 512 pp). "Stadia" statistics package was used for processing of the results.

Average values were compared using Student t-test. Seed germination in the control and experimental variants was compared using the test of agreement of frequencies with Z-statistics. The increase in germination rate, yield, height of seedlings and root length were calculated as a percentage relative to the control.

Effects of seed treatment with the new compounds of the pyrimidine-carboxylic acids range on seed germination, seedling height, yield of various plants and champignons compared to control, are presented in the tables 1-13.

As per the data provided in the Table 1 below, in case of experiment with tomatoes, the control group also involved Epin treatment. Epin, which is commercially available growth stimulator, had mild effect on the tomato seed germination (2.7% rate increase). The seeds treated with
2-amino-4-methylpyrimidine-5-yl-carboxylic acids demonstrated an increase in germination rate up to 3.6-4.4%, and those treated with the salts of
2-amino-4-methylpyrimidine-5-yl-carboxylic acids demonstrated an increase in germination rate up to 5.6-6.9%.

The height of tomato seedlings obtained from seeds treated with the 2-amino-4-methylpyrimidine-5-yl-carboxylic acids increased by 37.5-44.3% compared to the control group. When salts of 2-amino-4-methylpyrimidine-5-yl-carboxylic acids were used, this increase appeared to be 56.8-61.4%. When commercially available Epin stimulator was used, height increase compared to the control was only about 6.8%.

The maximum increase in yield was found when salts of 2-amino-4-methylpyrimidine-5-yl-carboxylic acids as per this invention were used, resulting in a 44-50% increase compared to the control.

Effect of aforementioned 2-amino-4-methylpyrimidine-5-yl-carboxylic acids and Epin resulted in yield increase of 32.1-36.9% and 10.7%, correspondingly.

A similar effect was also found for average fruit weight increase. Thus, with Epin, average weight of tomato increased for 3.5%, while use of 2-amino-4-methylpyrimidine-5-yl-carboxylic acids resulted in 5.1-5.6% increase, and use of 2-amino-4-methylpyrimidine-5-yl-carboxylic acids salts resulted in average fruit weight increase by 9.5-12.2%.

As can be seen from data presented in the Tables 1-13, yield increase for various crops treated with the 2-amino-4-methylpyrimidine-5-yl-carboxylic acids is within the range of 7.8-61.8%, depending on the crop, and when salts as per this invention are used, the effect is significantly higher compared to the related acid, and constitutes 25.1-101.8%, depending on the treated crop.

Increase in germination rage of seeds as the result of treatment with
2-amino-4-methylpyrimidine-5-yl-carboxylic acids is 0.8-14.4%, while in case of related salts it is much higher in each pair, resulting in 6.6-34.6% increase compared to the control group.

The length of seedlings increases by 9.8-94.7% with the use of 2-amino-4-methylpyrimidine-5-yl-carboxylic acids, while related salts resulted in a 21.6-121.1% increase.

### General method for obtaining of compounds of the formula (I)

N-substituted 2-amino-4-methylpyrimidine-5-yl-carboxylic acids or related esters are treated with related hydroxide of Na, K, Li, Mg, Ca, NH₃, or with carbonates/hydrocarbonates of Na, K, Li, Mg, Ca, NH₃, or with aliphatic and arylalkyl amine, in an equimolar ratio in aqueous media alone or mixed with the organic solvent. The preferred organic solvent is methanol or ethanol, or a combination of both. The interaction is preferably carried out under heating, with subsequent isolation of the obtained salt, or without isolation.

### Examples

### Experimental example No. 1 Preparation of the sodium salt of 2-benzylamino-4-methylpyrimidine-5-carboxylic acid.

To a mixture of 2.43 g (10 mmol) of the ethyl ester of 2-benzylamino-4-methylpyrimidine-5-carboxylic acid, 10 ml of ethanol, and 10 ml of water, 0.41 g (10.25 mmol) of sodium hydroxide in 10 ml of water was added. The reaction mixture was boiled under reflux until a clear solution was obtained. The volatile components were removed using a rotary evaporator. As a result, the sodium salt of 2-benzylamino-4-methylpyrimidine-5-carboxylic acid was obtained in quantitative yield.

### Experimental example No. 2 Preparation of potassium salt of 4-methyl-2-(piperidin-1-yl)pyrimidine-5-carboxylic acid.

To a mixture of 2.21 g (10 mmol) of the 4-methyl-2-(piperidin-1-yl)pyrimidine-5-carboxylic acid, 5 ml of methanol, and 15 ml of water, 1.02 g (10.20 mmol) of potassium hydroxide in 10 ml of water was added. The reaction mixture was agitated and heated (to a temperature of 50-60 °C) until a clear solution was obtained. The volatile components were removed using a rotary evaporator. As a result, the potassium salt of 4-methyl-2-(piperidin-1-yl)pyrimidine-5-carboxylic acid was obtained in quantitative yield.

### Experimental example No. 3 Preparation of ethanolamine salt of 4-methyl-2-(morpholin-4-yl)pyrimidine-5-carboxylic acid.

To a mixture of 2.28 g (10 mmol) of the 4-methyl-2-(morpholin-4-yl)pyrimidine-5-carboxylic acid, 10 ml of ethanol, and 10 ml of water, 0.62 g (10.15 mmol) of monoethanolamine was added. The reaction mixture was agitated and heated (to a temperature of 50-60 °C) until a clear solution was obtained. Further, the solution was cooled down to 0 °C, and kept for a while at this low temperature. The precipitate thus obtained was quickly filtered and washed with cold isopropanol. As a result, ethanolamine salt of 4-methyl-2-(morpholin-4-yl)pyrimidine-5-carboxylic acid was obtained in the 95% yield.

The physicochemical properties of preferred compounds obtained according to the above-described methods are presented below.

NMR 1H and 13C spectra were registered using Varian Inova 400 spectrometer (400 and 100 MHz, correspondingly).

### Example 1. Sodium salt of 2-benzylamino-4-methylpyrimidine-5-carboxylic acid.

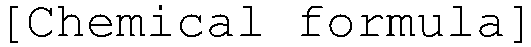

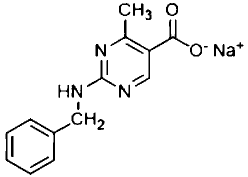

Melting point >300 °C. 1H NMR spectrum, DMSO-d6, δ, multiplicity; J, Hz: 2.58 (3H, s, CH3); 4.52 (2H, d, CH2N, J = 12.0); 7.16-7.28 (1H, m, CH-phenyl); 7.29-7.32 (4H, m, 4 CH-phenyl); 7.63 (1H, d, NH, J = 12.0); 8.65 (1H, s, CH-pyrimidine). 13C NMR spectrum, δ, multiplicity: 40.5, 44.4, 120.7, 125.8, 127.5, 128.5, 135.6, 141.2, 155.0, 161.7, 170.1.

### Example 2. Ethanolamine salt of 2-benzylamino-4-methylpyrimidine-5-carboxylic acid.

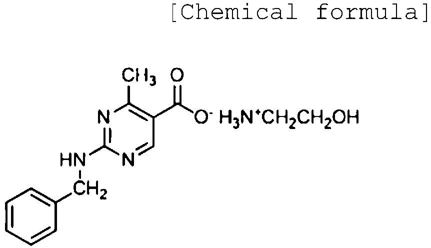

Amorphous substance. ¹H NMR spectrum, DMSO-d6, δ, multiplicity; J, Hz: 2.49-2.56 (3.9H, m, CH3 + DMSO); 2.81 (2H, t, CH2NH3, J = 6.0 Hz); 3.54-3.57 (3.5H, m, CH2OH + H2O); 4.52 (2H, d, CH2N, J = 12.0 Hz); 5.19 (1H, br s, NH3); 7.18-7.22 (1H, m, CH-phenyl); 7.25-7.30 (4H, m, 4 CH-phenyl); 7.71 (1H, br s, NH); 8.58 (1H, s, CH-pyrimidine). 13C NMR spectrum, δ, multiplicity: 40.5, 42.5, 44.3, 59.5, 119.9, 126.9, 127.5, 128.6, 140.9, 158.7, 161.9, 165.5, 170.1.

### Example 3. Potassium salt of 4-methyl-2-(piperidin-1-yl)pyrimidine-5-carboxylic acid.

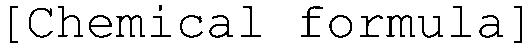

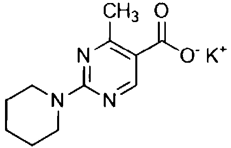

Melting point >300 °C. 1H NMR spectrum, DMSO-d6, δ, multiplicity; J, Hz: 1.43-1.51 (4H, m, 4 CH-piperidine); 1.57-1.62 (2H, m, 2CH-piperidine); 2.56 (3H, s, CH3); 3.70-3.73 (4H, m, 2CH2-piperidine); 8.60 (1H, s, CH-pyrimidine). 13C NMR spectrum, δ, multiplicity: 24.8, 24.9, 25.8, 40.5, 40.6, 121.9, 160.3, 160.5, 167.0, 169.1.

### Example 4. Propanolamine salt of 2-((4-methoxyphenyl)amino)-4-methylpyrimidine-5-carboxylic acid.

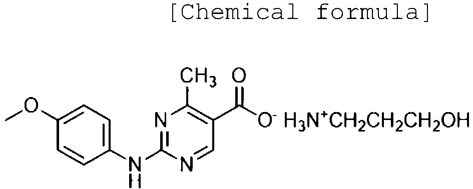

Amorphous substance. 1H NMR spectrum, DMSO-d6, δ, multiplicity; J, Hz: 1.71-1.75 (2H, m, CH2CH2CH2); 2.59 (3H, s, CH3); 2.80-2.84 (2H, m, CH2NH3); 3.48-3.53 (2H, m, CH2OH); 3.73 (3H, s, CH3O); 5.70 (4H, br s, NH3 + OH); 6.83-6.86 (2H, m, 2 CH-aryl); 7.69-7.72 (2H, m, 2 CH-aryl); 8.70 (1H, s, CH-pyrimidine); 9.41 (1H, s, NH). 13C NMR spectrum, δ, multiplicity: 24.5, 31.3, 37.2, 55.8, 58.6, 114.6, 120.9, 134.1, 154.4, 159.3, 160.3, 162.7, 167.1; 169.6.

### Example 5. Lithium salt of 4-methyl-2-(4-methylpiperazin-1-yl)pyrimidine-5-carboxylic acid.

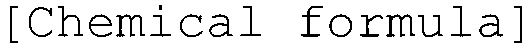

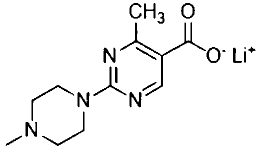

Melting point >300 °C. 1H NMR spectrum, DMSO-d6, δ, multiplicity; J, Hz: 2.36 (4H, br s, 2CH2-piperazine); 2.44 (3H, s, NCH3); 2.57 (3H, s, CH3); 3.69-3.72 (4H, m, 2CH2-piperazine); 8.62 (1H, s, CH-pyrimidine). 13C NMR spectrum, δ, multiplicity: 25.7, 43.4, 51.8, 54.7, 122.1, 160.3, 162.4, 166.8, 169.9.

### Example 6. Potassium salt of 4-methyl-2-((2,4-difluorophenyl)amino)pyrimidine-5-carboxylic acid.

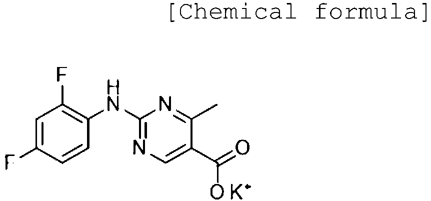

Melting point >300 °C. 1H NMR spectrum, D2O, δ, multiplicity; J, Hz: 2.33 (3H, s, CH3); 6.77-6.90 (2H, m, CH-arom.); 7.33-7.39 (1H, m, CH-arom.); 8.22 (1H, s, CH-pyrimidine). 13C NMR spectrum, D2O, δ, multiplicity; J, Hz: 22.5, 104.2 (br t, J = 25.7), 111.1, 111.3, 122.6, 127.1, 127.2, 158.3, 158.5 and 160.9 (CF), 159.3 and 161.0 (CF), 168.4, 173.5.

### Example 7. Sodium salt of 4-methyl-2-((4-methylphenyl)amino)pyrimidine-5-carboxylic acid.

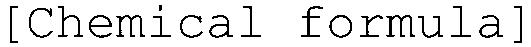

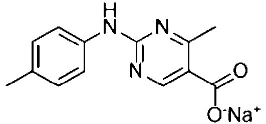

Melting point >300 °C. 1H NMR spectrum, D2O, δ, multiplicity; J, Hz: 2.09 (3H, s, CH3); 2.31 (3H, s, CH3); 6.99 (2H, d, CH-arom.); 7.11 (2H, d, CH-arom.); 8.22 (1H, s, CH-pyrimidine). 13C NMR spectrum, D2O, δ, multiplicity: 19.9, 22.7, 121.6, 129.3, 133.9, 135.4, 158.6, 158.9, 168.4, 173.5.

### Example 8. Lithium salt of 4-methyl-2-(phenethylamino)pyrimidine-5-carboxylic acid.

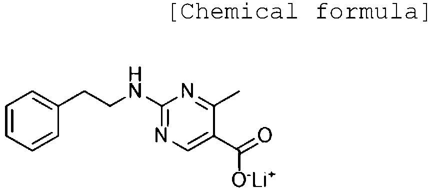

Melting point >300 °C. 1H NMR spectrum, D2O, δ, multiplicity; J, Hz: 2.24 (3H, s, CH3); 2.67 (2H, t, CH2, J = 6.6); 3.38 (2H, t, CH2, J = 6.6); 6.86-6.91 (3H, m, CH-arom.); 6.94-6.99 (2H, m, CH-arom.); 8.14 (1H, s, CH-pyrimidine). 13C NMR spectrum, D2O, δ, multiplicity: 22.6, 34.6, 42.1, 119.9, 126.2, 128.3, 128.7, 139.2, 158.7, 160.5, 168.5, 173.6.

### Example 9. Ethanolamine salt of 4-methyl-2-(morpholin-4-yl)pyrimidine-5-carboxylic acid.

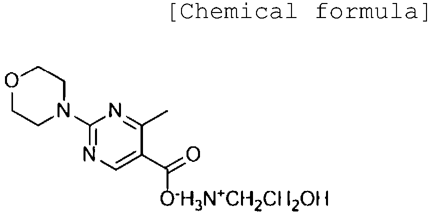

Melting point 218-221 °C. 1H NMR spectrum, D2O, δ, multiplicity; J, Hz: 2.33 (3H, s, CH3); 2.89-2.96 (2H, m, CH2N); 3.51-3.67 (10H, m, CH2O + 4 CH2-morpholine); 7.01-7.17 (2H, m, CH-arom.); 8.26 (1H, s, CH-pyrimidine) 13C NMR spectrum, D2O, δ, multiplicity: 22.9, 41.2, 44.2, 57.5, 66.2 120.3, 158.4, 160.3, 168.3, 173.9.

### Example 10. Phenethylamine salt of 4-methyl-2-(piperidin-1-yl)pyrimidine-5-carboxylic acid.

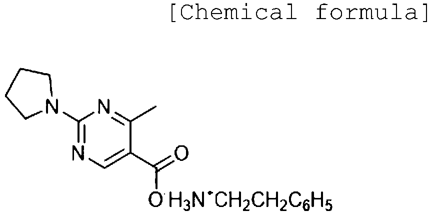

Melting point 139-143 °C. 1H NMR spectrum, D2O, δ, multiplicity; J, Hz: 1.67-1.78 (4H, m, 2 CH2-pyrrolidine); 2.29 (3H, s, CH3); 2.59-2.63 (2H, m, CH2); 2.99-3.08 (2H, m, CH2); 3.12-3.25 (4H, m, CH2CH2-pyrrolidine); 7.01-7.10 (3H, m, CH-arom.); 7.11-7.17 (2H, m, CH-arom.); 8.17 (1H, s, CH-pyrimidine). 13C NMR spectrum, D2O, δ, multiplicity: 22.7, 24.6, 32.6, 40.4, 46.8, 118.0, 126.9; 128.4; 128.7; 136.2; 157.9, 158.5; 168.4, 173.6.

### Example 11. Sodium salt of 2-(3,4-dihydroisoquinolin-2(1H)-yl)-4-methylpyrimidine-5-carboxylic acid.

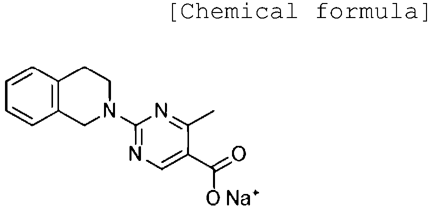

Melting point >300 °C. 1H NMR spectrum, D2O, δ, multiplicity; J, Hz: 2.32 (3H, s, CH3); 2.58 (2H, t, CH2, J = 5.8); 3.56 (2H, t, CH2, J = 5.8); 4.42 (2H, s, CH2); 6.87-6.98 (4H, m, CH-benz.); 8.20 (1H, s, CH-pyrimidine). 13C NMR spectrum, D2O, δ, multiplicity: 23.3, 27.9, 41.8, 45.9, 119.1, 126.1, 126.4, 128.0, 133.0, 134.8, 158.4, 158.6, 159.5, 168.5, 173.7.

### Example 12. Triethanolamine salt of 4-methyl-2-((3-methylphenyl)amino)pyrimidine-5-carboxylic acid.

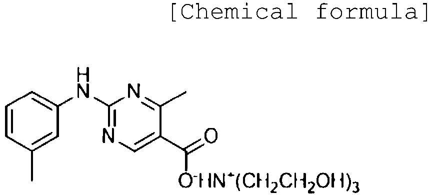

Amorphous substance. 1H NMR spectrum, D2O, δ, multiplicity; J, Hz: 2.12 (3H, c, CH3);
2.33 (3H, s, CH3); 2.71-2.76 (6H, m, N(CH2)3); 3.43-3.66 (6H, m, 3 CH2OH); 7.03-7.12 (3H, m, CH-arom.); 8.24 (1H, s, CH-pyrimidine) 13C NMR spectrum, D2O, δ, multiplicity: 20.5, 22.6, 55.5, 58.0, 118.4, 121.9, 122.2, 124.6, 128.9, 138.2, 139.2, 158.3, 158.8, 168.3, 173.5.

### Example 13. Triethanolamine salt of 2-benzylamino-4-methylpyrimidine-5-carboxylic acid.

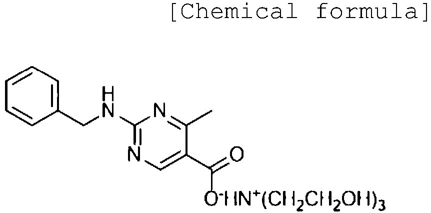

Amorphous substance. 1H NMR spectrum, D2O, δ, multiplicity; J, Hz: 2.30 (3H, s, CH3); 2.88-2.93 (6H, m, N (CH2)3); 3.59-3.63 (6H, m, 3 CH2OH); 4.36 (2H, s, CH2); 7.14-7.18 (5H, m, CH-arom.); 8.18 (1H, s, CH-pyrimidine). 13C NMR spectrum, D2O, δ, multiplicity: 22.6, 44.3, 55.4, 56.8, 120.7, 126.9, 127.1, 128.6, 138.8, 158.7, 160.7, 168.6, 173.8.

The use of the proposed water-soluble plant growth stimulators will significantly simplify application and increase the effectiveness of N-substituted 2-amino-4-methylpyrimidine-5-yl-carboxylic acid derivatives in agricultural practices.

The indicators of seed germination, seedling and root height, yield, and average fruit weight of various plant species and champignons after treatment with salts of 2-amino-4-methylpyrimidine-5-yl-carboxylic acids, as well as comparative data obtained from treatment with the corresponding acids, are presented in Tables 1-13.

Seed germination, seedling height, yield, and average weight of the Novichok tomato fruit after the treatment with known 2-amino-4-methylpyrimidine-5-yl-carboxylic acids vs. related new salts as per this invention.

**[Table 1]**

| Name of the compound | Germination rate of seeds, % | Height of seedlings at day 50 mm | Yield, kg/m² | Average fruit weight, g | Increase in height of seedling s compared to the control group, % |
|---|---|---|---|---|---|
| Water (control group) | 51.3 | 88 ± 2 | 8.4 ± 1 | 81.8 ± 1 | - |
| Epin 0.05% | 54.0 | 94 ± 2^{a} | 9.3 ± 1^{a} | 84.7 ± 1b | 6.8 |
| 2-benzylamino-4-methyl pyrimidine-5-carboxylic acid | 55.8 | 127 ± 2^{b} | 11.5 ± 2^{a} | 86.4 ± 2^{b} | 44.3 |
| 4-methyl-2-((2,4-difluorophenyl)ami no)pyrimidine-5-carboxylic acid | 55.3 | 124 ± 3^{b} | 11.0 ± 1^{a} | 85.2 ± 2^{b} | 40.9 |
| 4-methyl-2-(piperidin-1-yl)pyrimidine-5-carboxylic acid | 54.9 | 121 ± 2^{c} | 11.1 ± 2^{b} | 86.0 ± 2^{c} | 37.5 |
| 4-methyl-2-(phenethylamino)py rimidine-5-carboxylic acid | 54.2 | 122 ± 3^{c} | 11.5 ± 2^{c} | 84.9 ± 1^{b} | 38.6 |
| Sodium salt of 2-benzylamin-4-methyl pyrimidine-5-carboxylic acid | 57.5 | 142 ± 3^{c} | 12.6+2^{b} | 91.8+2^{b} | 61.4 |
| Ethanolamine salt of 2-benzylamin-4-methyl pyrimidine-5-carboxylic acid | 58.2 | 140 ± 3^{c} | 12.2 ± 2^{b} | 91.3 ± 2^{c} | 59.1 |
| Potassium salt of 4-methyl-2-((2,4-difluorophenyl)ami no)pyrimidine-5-carboxylic acid | 57.2 | 137 ± 2^{c} | 12.0 ± 1^{b} | 90.2 ± 1^{c} | 55.7 |
| Lithium salt of 4-methyl-2(phenethylamino) pyrimidine-5-carboxylic acid | 57.8 | 141 ± 1^{b} | 12.5 ± 2^{c} | 91.0 ± 2^{b} | 60.1 |
| Potassium salt of 4-methyl-2-(piperidin-1-yl)pyrimidine-5-carboxylic acid | 56.9 | 138 ± 2^{c} | 12.1 ± 2^{b} | 89.6 ± 2^{b} | 56.8 |

Seed germination rate, height of seedlings, yield and average fruit weight of common aubergine (Solanum melongena L.), Sirenivyy cultivar, after treatment with 2-amino-4-methylpyrimidine-5-yl-carboxylic acids vs. related new salts as per this invention.

**[Table 2]**

| Name of the compound | Germina tion rate of seeds, % | Seedling height after 50 days, mm | Yield, kg/m² | Yield increase compared to the control group, % | Average fruit weight, g | Increa se in height of seedli ngs COMPAR ED to the contro 1 group, % |
|---|---|---|---|---|---|---|
| Water (control group) | 45.3 | 74 ± 2 | 3.0 ± 1 | - | 198 ± 1 | - |
| 2-benzylamino-4-methylpyrimidine-5-carboxylic acid | 62.5 | 98 ± 2^{b} | 4.4 ± 2^{a} | 46.7 | 219 ± 2^{b} | 32.4 |
| 4-methyl-2-(4-methylpiperazin-1-yl)pyrimidine-5-carboxylic acid | 56.7 | 104 ± 2b | 4.5 ± 2^{b} | 50.0 | 225 ± 2^{c} | 40.5 |
| 4-methyl-2-((4-methylphenyl)amino )pyrimidine-5-carboxylic acid | 58.9 | 101 ± 2^{c} | 4.5 ± 1^{b} | 50.0 | 221 ± 2^{b} | 36.5 |
| Ethanolamine salt of 2-benzylamino-4-methylpyrimidine-5-carboxylic acid | 76.1 | 118 ± 2^{c} | 5.0+2^{b} | 66.7 | 258+2^{b} | 59.4 |
| Lithium salt of 4-methyl-2-(4-methylpiperazin-1-yl)pyrimidine-5-carboxylic acid | 79.4 | 115 ± 2^{c} | 5.1 ± 2^{c} | 70.0 | 261 ± 2^{b} | 54.4 |
| Sodium salt of 4- methyl-2-((4-methyl phenyl)amino)pyrim idine-5carboxylic acid | 77.6 | 117 ± 2^{b} | 5.0 ± 2^{c} | 66.7 | 262 ± 1^{b} | 58.1 |

Seed germination rate and height of seedlings of perennial Rhododendron Ledebourii (Rhododendron Ledebourii Pojarc.) after treatment with known 2-amino-4-methylpyrimidine-5-yl-carboxylic acids vs. related new salts as per this invention.

**[Table 3]**

| Name of the compound | Seed germinati on rate, % | Seedling height after 3 months, mm | Increase in height of seedlings compared to the control group, % | Seedling height after 6 months, mm | Increase in height of seedling s compared to the control group, % |
|---|---|---|---|---|---|
| Water (control group) | 56.3 | 19 ± 1 | - | 54 ± 1 | - |
| 2-benzylamino-4-methylpyrimidine-5-carboxylic acid | 55.9 | 29 ± 1^{b} | 65.5 | 76 ± 1^{b} | 38.2 |
| 4-methyl-2-(4-methylpiperazin-1-yl)pyrimidine-5-carboxylic acid | 69.4 | 37 ± 1^{b} | 94.7 | 75 ± 2^{b} | 33.9 |
| 4-methyl-2-(piperidin-1-yl)pyrimidine-5-carboxylic acid | 66.8 | 34 ± 2^{b} | 78.9 | 70 ± 1^{b} | 29.6 |
| Sodium salt of 2-benzylamino-4-methylpyrimidine-5-carboxylic acid | 79.2 | 39 ± 1^{b} | 105.3 | 80 ± 1^{b} | 48.1 |
| Triethanolamine salt of 2-benzylamino-4-methylpyrimidine-5-carboxylic acid | 79.0 | 41 ± 1^{b} | 115.8 | 81 ± 1^{c} | 50.0 |
| Lithium salt of 4-methyl-2-(4-methylpiperazin-1-yl)pyrimidine-5-carboxylic acid | 78.3 | 42 ± 1^{c} | 121.1 | 83+2^{b} | 53.7 |
| Potassium salt of 4-methyl-2-(piperidin-1-yl)pyrimidine-5-carboxylic acid | 81.0 | 41 ± 1^{c} | 115.8 | 78 ± 1^{c} | 44.4 |

Seeds germination rate and height of French marigolds (Tagetes patula L.), Krasnaya vishnya cultivar, after treatment with known 2-amino-4-methylpyrimidine-5-yl-carboxylic acids vs. related new salts as per this invention.

**[Table 4]**

| Name of the compound | Germinat ion rate of seeds, % | Seedling height after 50 days, mm | min-max, cm | Increase in height of seedlings compared to the control group, % |
|---|---|---|---|---|
| Water (control group) | 35.7 | 19 ± 1 | 16-22 | - |
| 2-((4-methoxyphenyl)amino)4-methylpyrimidine-5-carboxylic acid | 38.2 | 25 ± 1^{b} | 18-28 | 31. 6 |
| 4-methyl-2-(4-methylpiperazin-1-yl)pyrimidine-5-carboxylic acid | 43.3 | 27 ± 1^{b} | 19-30 | 42.1 |
| 4-methyl-2-(piperidin-1-yl)pyrimidine-5-carboxylic acid | 44.7 | 26 ± 2^{b} | 18-30 | 36.8 |
| 4-methyl-2-(phenethylamino)pyrimi dine-5-carboxylic acid | 39.5 | 28 ± 1^{b} | 19-31 | 47.4 |
| Lithium salt of 4-methyl-2(phenethylamino)pyrim idine-5-carboxylic acid | 49.7 | 32 ± 1^{b} | 25-37 | 68.4 |
| Propanolamine salt of 2-((4-methoxyphenyl)amino)-4-methylpyrimidine-5-carboxylic acid | 48.6 | 33 ± 1^{b} | 27-38 | 73.7 |
| Lithium salt of 4-methyl-2-(4-methylpiperazin-1-yl)pyrimidine-5-carboxylic acid | 50.5 | 32 ± 1^{c} | 25-35 | 68.4 |
| Potassium salt of 4-methyl-2-(piperidin-1-yl)pyrimidine-5-carboxylic acid | 47.1 | 35 ± 1^{c} | 28-43 | 82.2 |

Seed germination rate, height and yield of winter wheat (Triticum aestivum L.), Alekseich cultivar, after treatment with known 2-amino-4-methylpyrimidine-5-yl-carboxylic acids vs. related new salts as per this invention.

**[Table 5]**

| Name of the compound | Seed germinat ion rate, % | Height of seedling s after 30 days after sproutin g, cm | Increase in height of seedlings compared to the control group, % | Yield, 100 kg/ ha | Yield increase compared to control group, % |
|---|---|---|---|---|---|
| Water (control group) | 87.9 | 37 ± 0.2 | - | 58.1 ± 0.1 | |
| 2-((4-methoxyphenyl)amino)4-methylpyrimidine-5-carboxylic acid | 88.7 | 41 ± 0.2^{b} | 10.8 | 70.3 ± 0.1^{b} | 21.0 |
| 4-methyl-2-(4-methylpiperazin-1-yl)pyrimidine-5-carboxylic acid | 89.3 | 43 ± 0.2^{c} | 16.2 | 73.6 ± 0.1^{c} | 26.7 |
| 4-methyl-2-(piperidin-1-yl)pyrimidine-5-carboxylic acid | 90.4 | 42 ± 0.2^{b} | 13.5 | 73.8 ± 0.1^{b} | 27.0 |
| Propanolamine salt of 2-((4-methoxyphenyl)amino)-4-methylpyrimidine-5-carboxylic acid | 90.8 | 46 ± 0.2^{c} | 24.3 | 80.4 ± 0.1^{c} | 38.4 |
| Lithium salt of 4-methyl-2-(4-methylpiperazin-1-yl)pyrimidine-5-carboxylic acid | 93.1 | 48 ± 0.2^{c} | 29.7 | 83.5 ± 0.1^{b} | 43.7 |
| Potassium salt of 4-methyl-2-(piperidin-1-yl)pyrimidine-5-carboxylic acid | 96.6 | 45 ± 0.1^{c} | 21.6 | 85.6 ± 0.1^{b} | 47.3 |

Yield and average weight of the potato tuber (Solanum tuberosum), Zekura cultivar, after treatment with the known
2-amino-4-methylpyrimidine-5-yl-carboxylic acids vs. related new salts as per this invention.

**[Table 6]**

| Name of the compound | Yield, kg/m² | Average weight of tuber, kg | Yield increase compared to the control group, % |
|---|---|---|---|
| Water (control group) | 2.71 ± 0.2 | 0.064 ± 0.002 | - |
| 2-benzylamino-4-methylpyrimidine-5-carboxylic acid | 2.92 ± 0.2^{b} | 0.067 ± 0.002^{b} | 7.8 |
| 4-methyl-2-(morpholin-4-yl)pyrimidine-5-carboxylic acid | 2.91 ± 0.2^{c} | 0.067 ± 0.002^{b} | 7.4 |
| 4-methyl-2-(4-methylpiperazin-1-yl)pyrimidine-5-carboxylic acid | 2.98 ± 0.2^{b} | 0.068 ± 0.002^{b} | 10.1 |
| 4-methyl-2-[(2,4-difluorophenyl)amino] pyrimidine-5-carboxylic acid | 2.83 ± 0.1^{b} | 0.068 ± 0.001^{b} | 4.4 |
| Ethanolamine salt of 2-benzylamino-4-methylpyrimidine-5-carboxylic acid | 3.41 ± 0.2^{b} | 0.071 ± 0.002^{c} | 25.8 |
| Potassium salt of 4-methyl-2-[(2,4-difluorophenyl)amino] pyrimidine-5-carboxylic acid | 3.36 ± 0.2^{b} | 0.070 ± 0.001^{b} | 24.0 |
| Ethanolamine salt of 4-methyl-2-(morpholin- 4-yl)pyrimidine-5-carboxylic acid | 3.40 ± 0.2^{c} | 0.071 ± 0.001^{c} | 25.5 |
| Lithium salt of 4-methyl-2-(4-methylpiperazin-1-yl)pyrimidine-5-carboxylic acid | 3.39 ± 0.2^{b} | 0.069 ± 0.002^{c} | 25.1 |

Yield of sunflower seeds (Helianthus L.), Chakinskiy 100 cultivar, after treatment with known 2-amino-4-methylpyrimidine-5-yl-carboxylic acids vs. related new salts as per this invention.

**[Table 7]**

| Name of the compound | S, ha | Yield, 100 kg/ha | Yield increase compared to the control group, % |
|---|---|---|---|
| Water (control group) | 5 | 17.1 ± 0.2 | - |
| 2-benzylamino-4-methylpyrimidine-5-carboxylic acid | 5 | 23.0 ± 0.2^{b} | 35.3 |
| 4-methyl-2-(4-methylpiperazin-1-yl)pyrimidine-5-carboxylic acid | 5 | 23.9 ± 0.2^{b} | 39.8 |
| 4-methyl-2-(piperidin-1-yl)pyrimidine-5-carboxylic acid | 5 | 23.5 ± 0.2^{b} | 37.4 |
| Ethanolamine salt of 2-benzylamino-4-methylpyrimidine-5-carboxylic acid | 5 | 25.7 ± 0.2^{c} | 50.3 |
| Phenethylamine salt of 4-methyl-2-(piperidin-1-yl)pyrimidine-5-carboxylic acid | 5 | 26.2 ± 0.2^{b} | 53.2 |
| Lithium salt of 4-methyl-2-(4-methylpiperazin-1-yl)pyrimidine-5-carboxylic acid | 5 | 26.8 ± 0.2^{c} | 56.7 |

Yield of maize hybrid (Zea mays L.), Mixi cultivar, after treatment with known 2-amino-4-methylpyrimidine-5-yl-carboxylic acids vs. related new salts as per this invention.

**[Table 8]**

| Name of the compound | S, ha | Yield of dry clean grain, 100 kg/ha | Yield increase compared to the control group, % |
|---|---|---|---|
| Water (control group) | 15 | 55 ± 0.3 | - |
| 2-benzylamino-4-methylpyrimidine-5-carboxylic acid | 15 | 90 ± 0.2^{b} | 63.64 |
| 4-methyl-2-(piperidin-1-yl)pyrimidine-5-carboxylic acid | 15 | 87 ± 0.3^{b} | 58.18 |
| 2-((4-methoxyphenyl) amino) 4 -methylpyrimidine-5-yl-carboxylic acid | 15 | 89 ± 0.3^{b} | 61.82 |
| 4-methyl-2-(4-methylpyperazin-1-yl)pyrimidine-5-carboxylic acid | 15 | 83 ± 0.2^{c} | 50.91 |
| Sodium salt of 2-benzylamino-4-methyl pyrimidine-5-carboxylic acid | 15 | 111 ± 0.1^{c} | 101.82 |
| Ethanolamine salt of 2-benzylamino-4-methyl pyrimidine-5-carboxylic acid | 15 | 104 ± 0.2^{a} | 89.09 |
| Potassium salt of 4-methyl-2-(piperidin-1-yl)pyrimidine-5-carboxylic acid | 15 | 98 ± 0.1^{c} | 78.18 |
| Propanolamine salt of 2- ((4-methoxyphenyl)amino)-4-methylpyrimidine-5-carboxylic acid | 15 | 105 ± 0.3^{b} | 90.91 |
| Lithium salt of 4-methyl-2-(4-methylpyperazin-1-yl)pyrimidine-5-carboxylic acid | 15 | 103 ± 0.2 | 87.27 |

Yield and average fruit weight for zucchini (Cucurbita pepo subsp. pepo), Tintoretto cultivar, after treatment with known
2-amino-4-methylpyrimidine-5-yl-carboxylic acids vs. related new salts as per this invention.

**[Table 9]**

| Name of the compound | Fruit weight, kg | Increase in fruit weight compared to the control group, % | Yield, kg/m² | Increase in yield, compared to the control group, % |
|---|---|---|---|---|
| Water (control group) | 1.65 ± 0.03 | - | 7.50 ± 0. 03 | - |
| 2-benzylamino-4-methylpyrimidine-5-yl-carboxylic acid | 1.78 ± 0.04^{a} | 7.88 | 8.34 ± 0. 04^{a} | 11.21 |
| 2-((4-methoxyphenyl) amino)-4-methylpyrimidine-5-carboxylic acid | 1.84 ± 0.02^{b} | 11.52 | 8.55 ± 0. 04a | 14.00 |
| 4-methyl-2-(4-methylpyperazin-1-yl)pyrimidine-5-carboxylic acid | 1.81 ± 0.02^{b} | 9.70 | 8.46 ± 0. 03b | 12.80 |
| Sodium salt of 2-benzylamino-4-methylpyrimidine-5-carboxylic acid | 2.18 ± 0.02^{c} | 32.12 | 10.23 ± 0 .02^{b} | 36.40 |
| Triethanolamine salt of 2-benzylamino-4-methylpyrimidine-5-yl-carboxylic acid | 2.15 ± 0.03^{b} | 30.3 | 9.87 ± 0. 03^{b} | 31.6 |
| Propanolamine salt of 2-((4-methoxyphenyl)amino)-4-methylpyrimidine-5-carboxylic acid | 2.08 ± 0.03^{b} | 26.06 | 9.73 ± 0. 03a | 29.73 |
| Lithium salt of 4-methyl-2-(4-methylpiperazin-1-yl)pyrimidine-5-carboxylic acid | 2.12 ± 0.04^{a} | 28.48 | 9.82 ± 0. 04a | 30.93 |

Germination rate and seedling length of pea (Pisum L.), Orlus cultivar
after treatment with known 2-amino-4-methylpyrimidine-5-yl-carboxylic acids vs. related new salts as per this invention.

**[Table 10]**

| Name of the compound | Seed germination rate, % | Increase in germination rate compared to the control group, % | Length of seedlings at the day 10, cm | Increase in seedling length, compared to the control group, % |
|---|---|---|---|---|
| Water (control group) | 89.0 | - | 8.25 ± 0. 04 | - |
| 2-((4-methoxyphenyl) amino) 4-methylpyrimidine-5-carboxylic acid | 93.3 | 4.8 | 11.26 ± 0 .03^{b} | 36.48 |
| 4-methyl-2-(4-methylpiperazin-1-yl)pyrimidine-5-carboxylic acid | 93.4 | 4.9 | 11.54 ± 0 .04^{a} | 39.87 |
| 4-methyl-2-(morpholin-4-yl)pyrimidine-5-carboxylic acid | 93.0 | 4.5 | 11.31 ± 0 .02^{b} | 37.1 |
| 4-methyl-2-(piperidin-1-yl)pyrimidine-5-carboxylic acid | 92.9 | 4.4 | 11.22 ± 0 .03^{c} | 36.0 |
| Ethanolamine salt of 4-methyl-2-(morpholin-4-yl)pyrimidine-5-carboxylic acid | 94.9 | 6.6 | 12.55 ± 0 .03^{b} | 52.1 |
| Propanolamine salt of 2-((4-methoxyphenyl) amino)4-methylpyrimidine-5-carboxylic acid | 94.8 | 6.5 | 12.42 ± 0 .02^{c} | 50.55 |
| Lithium salt of 4-methyl-2-(4-methylpiperazin-1-yl)pyrimidine-5-carboxylic acid | 95.2 | 7.0 | 12.94 ± 0 .03^{c} | 56.85 |
| Phenethylamine salt of 4-methyl-2-(piperidin-1-yl)pyrimidine-5-carboxylic acid | 94.5 | 6.2 | 12.88 ± 0 .03^{c} | 56.1 |

Yield and head weight of white cabbage (Brassica oleracea L.), Podarok cultivar, after treatment with known 2-amino-4-methylpyrimidine-5-yl-carboxylic acids vs. related new salts as per this invention.

**[Table 11]**

| Name of the compound | Yield, 100 kg/ha | Yield increase compared to the control group, % | Yield, commercial, 100 kg/ha | Increase in commercial yield, compared to the control group, % | Average head weight, kg |
|---|---|---|---|---|---|
| Water (control group) | 48.3 ± 0. 04 | - | 46.1 ± 0. 02 | - | 2.11 ± 0. 01 |
| 2-benzylamino-4-methylpyrimidine-5-carboxylic acid | 54.8 ± 0. 02^{b} | 13.5 | 52.4 ± 0. 02^{b} | 13.7 | 2.25 ± 0. 01^{b} |
| 4-methyl-2-(4-methylpiperazin-1-yl)pyrimidine-5-carboxylic acid | 55.0 ± 0. 02^{b} | 13.9 | 54.3 ± 0. 01^{c} | 17.8 | 2.30 ± 0. 01^{b} |
| 4-methyl-2-(piperidin- 1-yl)pyrimidine-5-carboxylic acid | 56.7 ± 0. 02^{b} | 17.4 | 54.2 ± 0. 02^{b} | 17.6 | 2.34 ± 0. 01^{b} |
| Ethanolamine salt of 2-benzylamino-4-methylpyrimidine-5-yl-carboxylic acid | 61.6+0.02^{b} | 27.5 | 58.9+0.02^{b} | 27.8 | 2.47 ± 0. 01^{b} |
| Lithium salt of 4-methyl-2-(4-methylpiperazin-1-yl)pyrimidine-5-carboxylic acid | 69.5 ± 0. 02^{c} | 43.9 | 66.4 ± 0. 02^{c} | 44.0 | 2.54 ± 0. 01^{b} |
| Potassium salt of 4-methyl-2-(piperidin-1-yl)pyrimidine-5-carboxylic acid | 64.9 ± 0. 02^{b} | 34.4 | 62.2 ± 0. 02^{c} | 34.9 | 2.51 ± 0. 01^{b} |

Yield and fruit weight of sweet pepper (Capsicum annuum L.), Flamingo cultivar, after treatment with known 2-amino-4-methylpyrimidine-5-yl-carboxylic acids vs. related new salts as per this invention.

**[Table 12]**

| Name of the compound | Fruit weight, | Increase in fruit weight compared to the control group, | Yield, kg/m² | Yield increase compared to the control group, |
|---|---|---|---|---|
| | g | % | | % |
| Water (control group) | 89.76 ± 0.02 | - | 4.38 ± 0.03 | - |
| 2-benzylamino-4-methylpyrimidine-5-carboxylic acid | 99.58 ± 0.0 3^{b} | 10.94 | 5.62 ± 0. 01^{c} | 28.31 |
| 2-((4-methoxyphenyl)amino)-4-methylpyrimidine-5-carboxylic acid | 103.72 ± 0. 02^{c} | 15.55 | 6.04 ± 0. 02^{b} | 37.90 |
| 2-(3,4-dihydroisoquinolin-2(1H)-yl) 4-methylpyrimidine-5-yl-carboxylic acid | 98.93 ± 0.0 3^{c} | 10.2 | 5.93 ± 0. 01^{c} | 35.4 |
| Sodium salt of 2-benzylamino-4-methylpyrimidine-5-yl-carboxylic acid | 128.14 ± 0. 02^{b} | 42.76 | 7.93 ± 0. 02^{b} | 81.05 |
| Sodium salt of 2-(3,4-dihydroisoquinolin-2(1H)-yl)-4-methylpyrimidine-5-yl-carboxylic acid | 127.89 ± 0. 02^{b} | 42.5 | 7.80 ± 0. 02^{c} | 78.1 |
| Propanolamine salt of 2-((4-methoxyphenyl)amino)-4-methylpyrimidine-5-carboxylic acid | 126.53 ± 0. 03^{b} | 40.96 | 7.85 ± 0. 01^{b} | 79.22 |

Yield of be-spore white button mushrooms, after treatment with known 2-amino-4-methylpyrimidine-5-yl-carboxylic acids vs. related new salts as per this invention.

**[Table 13]**

| Name of the compound | S, m² | Yield, kg/m² | Yield increase compared to the control group, % |
|---|---|---|---|
| Water (control group) | 2 | 4.8 ± 0.2^{a} | - |
| 2-benzylamino-4-methylpyrimidine-5-carboxylic acid | 2 | 5.4 ± 0.2^{a} | 12.5 |
| 4-Methyl-2-[(3-methylphenyl)amino]pyr imidine-5-carboxylic acid | 2 | 5.2 ± 0.3^{c} | 8.0 |
| Sodium salt of 2-benzylamino-4-methylpyrimidine-5-carboxylic acid | 2 | 6.1 ± 0.2a | 27.0 |
| Triethanolamine salt of 4-methyl-2-((3-methylphenyl)amino)pyr imidine-5-carboxylic acid | 2 | 5.9 ± 0.3^{c} | 22.9 |

| | | | |
|---|---|---|---|
| [0130] ^{a} - The differences with the control group are significant (P < 0.05); [0131] ^{b} - The differences with the control group are significant (P < 0.01); [0132] ^{c} - The differences with the control group are significant (P < 0.001). | | | |

## Claims

1. Compound which is a salt of N-substituted 2-amino-4-methylpyrimidine-5-yl-carboxylic acids corresponding to the general formula (I) 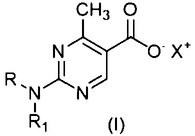
where X⁺ shall be selected from cations Na⁺, K⁺, Li+, ½ Mg²⁺, ½ Ca+, NH₄⁺, H₃N⁺CH₂CH₂OH, H₃N⁺CH₂CH₂CH₂OH, HN⁺(CH₂CH₂OH)₃ H₃N⁺CH₂CH₂C₆H₅, or
ANH₃⁺, A₂NH₂⁺, A₃NH⁺,
A is C₁-C₁₂ alkyl, C₆-C₁₂a ryl, C₅-C₁₂ heterocyclyl, C₅-C₁₂ heteroaryl, where heterocyclyl and heteroaryl compounds contain 1 to 3 hetero atoms, which can be N, O or S,
while each A may be optionally substituted by one or several groups, which independently can be selected from halogen, amino-, thio- or hydroxy-,
R = hydrogen,
R₁ means C₁-C₆ alkyl;
phenyl, optionally substituted with one or more substituents selected from (C₁-C₄)alkyl, methoxy, ethoxy, halogen; phenyl-(C₁-C₄) alkyl-;
or R+R₁ together with the nitrogen atom mean pyrrolidine, hydroquinoline, hydroisoquinoline, piperidine, morpholine, piperazine, optionally substituted by one or two substituting groups selected from halogen, -(C₁-C₄)alkyl or C₆-C₁₂ aryl.

2. Compound according to Claim 1, where R₁ is methyl, ethyl, propyl, phenyl, optionally substituted by one or two substituting groups selected from methyl, ethyl, methoxy group, halogen; and phenyl-(C₁-C₂)alkyl-.

3. Compound according to Claim 1, where R+R₁ together with the nitrogen atom mean pyrrolidine, hydroquinoline, hydroisoquinoline, piperidine, morpholine, piperazine, optionally substituted by or two substituting groups selected from methyl, ethyl, halogen and phenyl.

4. Compound according to Claim 1, where the cation is either Na⁺, or K⁺, Li₊, ½ Mg²⁺, ½Ca⁺, NH₄⁺, H₃N⁺CH₂CH₂OH, H₃N⁺CH₂CH₂CH₂OH, H₃N⁺CH₂CH₂C₆H₅, HN⁺(CH₂CH₂OH)₃.

5. Compound according to Claim 1, where compound is selected from the group including salts of 2-(2-phenylethyl)amino-4-methylpyrimidine-5-carboxylic acid,
4-methyl-2-(pyrrolidin-1-yl)pyrimidine-5-carboxylic acid,
4-methyl-2-(N-methylpiperazin-1-yl)pyrimidine-5-carboxylic acid,
4-methyl-2-((4-methoxyphenyl)amino)pyrimidine-5-carboxylic acid,
2-benzylamino-4-methylpyrimidine-5-carboxylic acid,
2-(3,4-dihydroisoquinolin-2(1H)-yl)-4-methylpyrimidine-5-carboxylic acid,
4-methyl-2-((2.4-difluorophenyl)amino)pyrimidine-5-carboxylic acid,
4-methyl-2-((4-methylphenyl)amino)pyrimidine-5-carboxylic acid,
4-methyl-2-(morpholin-4-yl)pyrimidine-5-carboxylic acid,
4-Methyl-2-(piperidin-1-yl)pyrimidine-5-carboxylic acid,
4-methyl-2-((3-methylphenyl)amino)pyrimidine-5-carboxylic acid.

6. Compound according to Claim 5, where salt is potassium salt, ethanolamine salt, sodium salt, propanolamine salt, lithium salt, phenethylamine salt, or triethanolamine salt.

7. Compound according to Claim 1, selected from sodium salt of
2-benzylamino-4-methylpyrimidine-5-carboxylic acid, potassium salt of 4-methyl-2-(piperidin-1-yl)pyrimidine-5-carboxylic acid, ethanolamine salt of
4-methyl-2-(morpholin-4-yl)pyrimidine-5-carboxylic acid, sodium salt of 2-benzylamino-4-methylpyrimidine-5-carboxylic acid, ethanolamine salt of
2-benzylamino-4-methylpyrimidine-5-carboxylic acid, potassium salt of 4-methyl-2-(piperidin-1-yl)pyrimidine-5-carboxylic acid, propanolamine salt of
2-((4-methoxyphenyl)amino)-4-methylpyrimidine-5-carboxylic acid, lithium salt of
4-methyl-2-(4-methylpiperazin-1-yl)pyrimidine-5-carboxylic acid, potassium salt of
4-methyl-2-((2,4-difluorophenyl)amino)pyrimidine-5-carboxylic acid, sodium salt of
4-methyl-2-((4-methylphenyl)amino)pyrimidine-5-carboxylic acid, lithium salt of 4-methyl-2-(phenethylamino)pyrimidine-5-carboxylic acid, ethanolamine salt of
4-methyl-2-(morpholin-4-yl)pyrimidine-5-carboxylic acid, phenethylamine salt of
4-methyl-2-(piperidin-1-yl)pyrimidine-5-carboxylic acid, sodium salt of 2-(3,4-dihydroisoquinolin-2(1H)-yl)-4-methylpyrimidine-5-carboxylic acid, triethanolamine salt of 4-methyl-2-((3-methylphenyl)amino)pyrimidine-5-carboxylic acid, and triethanolamine salt
2-benzylamino-4-methylpyrimidine-5-carboxylic acid.

8. Method of salt preparation, according to any of the Claims 1-7, involving contact in aqueous and/or organic solvent media between N-substituted 2-amino-4-methylpyrimidine-5-yl-carboxylic acid or related ester and solution of related hydroxide of Na, K, Li, Mg, Ca, NH₃ with subsequent isolation of the salt.

9. Method according to Claim 8, where contact is performed in aqueous media and/or in organic solvent, selected from ethanol, methanol or their combination.

10. Method according to Claim 8, where contact is performed under heating.

11. Method of salt preparation, according to any of the Claims 1-7, involving contact in aqueous and/or organic solvent media between N-substituted 2-amino-4-methylpyrimidine-5-yl-carboxylic acid or related ester and carbonate or hydrocarbonate of Na, K, Li, Mg, Ca, NH₃ with subsequent isolation of salt.

12. Method of salt preparation, according to any of the Claims 1-7, involving contact in aqueous and/or organic solvent media between N-substituted 2-amino-4-methylpyrimidine-5-carboxylic acid or corresponding ester and aliphatic or aromatic amines, with subsequent isolation of the obtained salt.

13. Method according to Claim 12, where contact is performed in aqueous media and/or in organic solvent, selected from ethanol, methanol or their combination.

14. Method according to Claim 13, where contact is performed under heating.

15. Use of compound according to any of Claims 1-7 for stimulation of plant or fungal growth.

16. The use according to Claim 15, where stimulation of plant or fungal growth is selected from stimulation of germination energy, stimulation of seed germination, stimulation of biometric parameters of seedlings, stimulation of root formation, and stimulation of yield of a wide range of agricultural crops.

17. The use according to Claim 15, where stimulation of growth is performed during pre-sowing seeds treatment and then on any subsequent stage of the crop development.

18. The use according to Claim 15, wherein the plant or fungus is selected from the group consisting of tomato, aubergine, rhododendron, marigold, wheat, potato, sunflower, corn, zucchini, pea, cabbage, pepper, and white button mushroom.
